(19) Europäisches Patentamt
European Patent Office
Office européen des brevets

(11) Veröffentlichungsnummer: **0 034 292**
**B1**

(12) # EUROPÄISCHE PATENTSCHRIFT

(45) Veröffentlichungstag der Patentschrift: **23.11.83**

(51) Int. Cl.³: **C 07 C 99/00,** C 07 C 101/54, C 07 C 103/46

(21) Anmeldenummer: **81100708.7**

(22) Anmeldetag: **31.01.81**

(54) **Verfahren zur Herstellung von Anthranilsäurederivaten.**

(30) Priorität: **06.02.80 US 119078**

(43) Veröffentlichungstag der Anmeldung: **26.08.81 Patentblatt 81/34**

(45) Bekanntmachung des Hinweises auf die Patenterteilung: **23.11.83 Patentblatt 83/47**

(84) Benannte Vertragsstaaten: **AT BE CH DE FR GB IT LI NL**

(56) Entgegenhaltungen:
**DE - A - 2 526 046**
**GB - A - 1 275 368**

**Chemical Abstracts Band 86, Nr. 11, 14 März 1977 Columbus, Ohio, USA L. CASSAR et al., «The use of phase-transfer catalysis in palladium-catalyzed carbonylation of organic halides» Seite 488, Spalte 1, Abstract Nr. 70973u**
**Chemical Abstracts Band 82, Nr. 1, 6 Januar 1975 Columbus, Ohio, USA A. SCHOENBERG et al. «Palladium-catalyzed carboalkoxylation of aryl, benzyl, and vinylic halides» Seite 338, Spalte 1, Abstracts Nr. 3922f**
**Houben-Weyl «Methoden der organischen Chemie», 4. Auflage, Band XI/1 1957, GEORG THIEME VERLAG, Stuttgart**

(73) Patentinhaber: **F. HOFFMANN-LA ROCHE & CO. Aktiengesellschaft, CH-4002 Basel (CH)**

(72) Erfinder: **Tilley, Jefferson Wright, 19 Evergreen Drive, North Caldwell New Jersey 07006 (US)**
Erfinder: **Valentine, Donald, Jr., 2865 Homestead Road Apartment 5, Santa Clara California 95051 (US)**

(74) Vertreter: **Kellenberger, Marcus, Dr. et al, Grenzacherstrasse 124 Postfach 3255, CH-4002 Basel (CH)**

**EP 0 034 292 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

# Verfahren zur Herstellung von Anthranilsäurederivaten

Die vorliegende Erfindung betrifft ein Verfahren zur Herstellung von Anthranilsäurederivaten der allgemeinen Formel

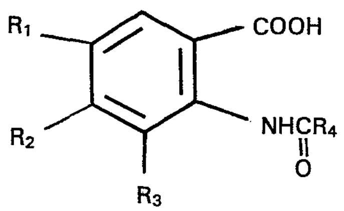

I

worin $R_1$, $R_2$ und $R_3$ unabhängig voneinander je Wasserstoff, niederes Alkyl, niederes Alkoxy, Carboxyl, Chlor, Fluor oder gegebenenfalls durch niederes Alkyl oder niederes Alkoky substituiertes Phenyl und $R_4$ niederes Alkyl oder Phenyl bedeuten,
gemäss welchem Kohlenmonoxyd in einem wässrigen Reaktionsmedium, welches ein Trialkylamin und einen Katalysator, gebildet aus Palladium und einem tertiären Phosphin, enthält, mit einer Verbindung der allgemeinen Formel

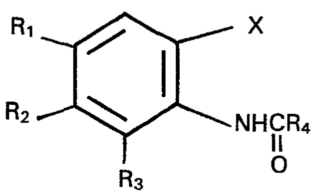

II

worin X Brom oder Jod bedeutet und $R_1$, $R_2$, $R_3$ und $R_4$ die oben angegebene Bedeutung besitzen,
umsetzt.

Die Verbindungen der obigen Formel I können in Gegenwart einer Base zu den entsprechenden Anthranilsäuren der allgemeinen Formel

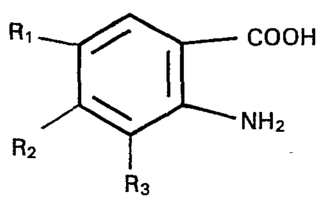

III

worin $R_1$, $R_2$ und $R_3$ die oben angegebene Bedeutung besitzen,
hydrolysiert werden.

Der in dieser Beschreibung verwendete Ausdruck «niederes Alkyl» betrifft geradkettige und verzweigtkettige Kohlenwasserstoffgruppen mit 1-7 Kohlenstoffatomen, wie Methyl, Äthyl, Propyl, Isopropyl, Butyl, Isobutyl, sec.-Butyl, tert.-Butyl, Neopentyl, Pentyl, Heptyl und dergleichen. Der Ausdruck «niederes Alkoxy» betrifft Alkyläthergruppen, in denen die Alkylreste die oben angegebene Bedeutung besitzen, wie Methoxy, Äthoxy, Propoxy, Isopropoxy, Butoxy, Pentoxy und dergleichen.

In der vorliegenden Beschreibung sind alle Temperaturen in Celsiusgraden angegeben. Sofern nicht anders angegeben handelt es sich bei allen Prozent- und Verhältnis-Angaben um Gewichtsangaben.

Bevorzugte Verbindungen der obigen Formeln I und III sind solche, worin $R_1$ niederes Alkyl oder niederes Alkoxy und $R_4$ niederes Alkyl bedeutet. Die Verbindungen der Formeln I und III sind stabile Handelsprodukte, welche beispielsweise nützliche Zwischenprodukte in der Herstellung von pharmazeutischen Wirkstoffen sind. Insbesondere sind die Verbindungen der Formeln I und III nützliche Zwischenprodukte bei der Herstellung von substituierten Pyridochinazolinverbindungen, welche ihrerseits wertvolle Antiallergika darstellen.

Die katalytische Carbonylierung von Arylhalogeniden unter Verwendung von komplexen Palladiumkatalysatoren zur Bildung entsprechender Benzoesäuren ist zwar allgemein bekannt, doch verläuft die Carbonylierung von 2-Haloanilinen, wie von 2-Bromanilin, unter Verwendung von komplexen Palladiumkatalysatoren mit relativ langsamer Reaktionsgeschwindigkeit, und es wird der Einsatz von relativ hohen Mengen Katalysator notwendig, um gute Ausbeuten zu erhalten.

J. Organometal. Chem., *121*, C55-56 (1976) beschreibt eine Methode zur Herstellung von Benzoesäuren in Form ihrer Natriumsalze. Die offenbarte Methode umfasst die Carbonylierung der entsprechenden aromatischen Halogenide in wässriger Natriumhydroxydlösung unter starkem Rühren mit einer Xylollösung eines Triphenylphosphinmetallkomplexes. Diese Methode ist eher unzweckmässig, insofern als zwei nicht miteinander mischbare Lösungsmittel zum Einsatz kommen, welche nach beendeter Reaktion getrennt werden müssen.

J. Org. Chem., *39*, 3318-3326 (1974) beschreibt eine Methode zur Herstellung von Benzoesäureestern durch Carbonylierung entsprechender aromatischer Halogenide in Gegenwart eines Palladiumtriphenylphosphinkatalysators in Alkohol. Falls die Herstellung der entsprechenden Säure erwünscht ist, ist jedoch ein zusätzlicher Schritt zur Überführung des Esters notwendig.

Im Hinblick auf die Schwierigkeiten, welche bei Palladium-katalysierten Carbonylierungsreaktionen von 2-Haloanilinen auftreten, war es unerwartet, dass die Carbonylierung von 2-Haloaniliden der obigen Formel II in ausgezeichneten Ausbeuten und guten Reaktionsgeschwindigkeiten unter Verwendung relativ geringer Mengen Katalysator abläuft.

Die Verbindungen der obigen Formel II sind bekannt oder können in bekannter Weise hergestellt werden. So können sie beispielsweise dadurch erhalten werden, dass man eine Verbindung der allgemeinen Formel

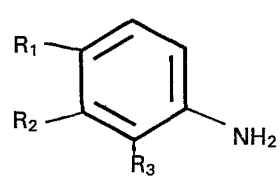

IV

worin $R_1$, $R_2$ und $R_3$ die oben angegebene Bedeutung besitzen,

mit einer Säure der Formel $R_4COOH$ umsetzt und anschliessend das erhaltene Produkt der allgemeinen Formel

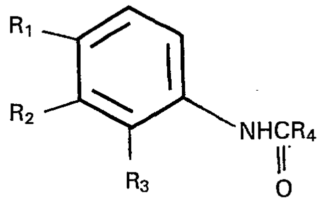

V

worin $R_1$, $R_2$, $R_3$ und $R_4$ die oben angegebene Bedeutung besitzen,
mit einem Halogenierungsmittel umsetzt. So kann beispielsweise Cumidin, d.h. Isopropylanilin (überwiegend das para-Isomere), mit Essigsäure zum 4-Isopropylacetanilid umgesetzt werden, welches seinerseits mit Brom zum entsprechenden 2-Brom-4-isopropylacetanilid umgesetzt werden kann.

Beim in der Carbonylierungsreaktion eingesetzten Trialkylamin handelt es sich um ein Tri-niederes-alkylamin, vorzugsweise Triäthylamin und Tri-n-butylamin.

Wie bereits erwähnt, wird in der Carbonylierungsreaktion ein Katalysator verwendet, welcher aus Palladium und einem tertiären Phosphin gebildet ist. Vorzugsweise wird als tertiäres Phosphin ein Triarylphosphin, besonders bevorzugt Triphenylphosphin, verwendet. Andere geeignete tertiäre Phosphine umfassen Alkyldiarylphosphine, wie Methyldiphenylphosphin. Der Katalysator wird zweckmässig der Reaktion in Form einer Verbindung zugeführt, welche aus Palladium und dem tertiären Phosphin erhalten wird. Beispielsweise kann der Katalysator dem Reaktionsgemisch als $PD[P(C_6H_5)_3]_2Cl_2$, $Pd[P(C_6H_5)_3]_2Br_2$ oder $Pd[P(C_6H_5)_3]_4$ zugegeben werden. Gemäss einer alternativen Reaktionsführung kann das tertiäre Phosphin und das Palladium auch getrennt dem Reaktionsgemisch beigefügt werden. In diesem Falle kann das Palladium in Form eines im Reaktionsmedium löslichen Salzes zugegeben werden, beispielsweise Palladiumchlorid, Palladiumnitrat, Palladiumacetat, und dergleichen.

Das molare Verhältnis von tertiärem Phosphin zu Palladium beträgt in der Regel mindestens 2:1. Es ist jedoch bevorzugt, dem Reaktionsgemisch das tertiäre Phosphin im Überschuss zuzuführen, d.h. in einem Verhältnis, welches grösser als 2:1 ist. Beispielsweise enthält ein Reaktionsgemisch ungefähr 8 mg $Pd[(C_6H_5)_3]_2Cl_2$ und 40 mg Triphenylphosphin. Obwohl keine strenge obere Grenze für das Verhältnis von tertiärem Phosphin zu Palladium existiert, bringt ein Verhältnis, das grösser als 5:1 ist, keine weiteren Vorteile.Pro Mol Katalysator, basierend auf der Molmenge von Palladium im Katalysator, werden in der Regel 100 bis 10 000 Mol Substrat, d.h. Verbindung der obigen Formel II, vorzugsweise etwa 250 bis 3000 Mol, eingesetzt.

In der Carbonylierungsreaktion wird pro Mol an Verbindung der Formel II mindestens je 1 Mol Kohlenmonoxyd, Wasser und Trialkylamin benötigt. Erwünschtenfalls können sie aber auch im molaren Überschuss eingesetzt werden.

Zusätzlich zu den vorstehend erwähnten Komponenten der Reaktionsmischung kann auch jedes inerte Lösungsmittel im Reaktionsgemisch anwesend sein, welches mit den übrigen Komponenten kompatibel ist, wie Toluol oder Dioxan.

Da der Katalysator durch die Gegenwart von Sauerstoff angegriffen wird, wird die Carbonylierungsreaktion unter im wesentlichen sauerstofffreien Bedingungen durchgeführt. Die Temperatur der Reaktion ist nicht kritisch, und die Reaktion verläuft zufriedenstellend bei Temperaturen zwischen etwa 80 und 200°.

Zweckmässig wird die Carbonylierungsreaktion so durchgeführt, dass man ein geeignetes Reaktionsgefäss mit den geeigneten Mengen an Verbindung der Formel II und Katalysator belädt, die Luft aus dem Reaktionsgefäss entfernt, beispielsweise durch Ersetzen mit einem Inertgas, wie Argon, und schliesslich das Trialkylamin, das Wasser und das Kohlenmonoxyd in das Reaktionsgefäss einbringt. Danach wird der Inhalt des Reaktionsgefässes auf die erwünschte Reaktionstemperatur erhitzt, und zwar so lange, bis die Reaktion beendet ist. Das Reaktionsprodukt, d.h. die erhaltene Verbindung der obigen Formel I, kann nach konventionellen Methoden, wie Kristallisation aus einem geeigneten Lösungsmittel, isoliert werden.

Die Verbindung der Formel I kann in Gegenwart einer Base zur Anthranilsäure der Formel III hydrolysiert werden. Geeignete Basen sind Alkalimetallhydroxyde, wie Natriumhydroxyd. Die Temperatur der Hydrolyse ist nicht kritisch, und die Reaktion verläuft zufriedenstellend bei Temperaturen zwischen etwa 20 und 150°. Das Endprodukt kann nach konventionellen Methoden durch Kristallisation aus einem geeigneten Lösungsmittel isoliert werden.

Die nachfolgenden Beispiele 3-14, 16, 18 und 19 illustrieren die Erfindung.

*Beispiel 1*

100 g (0,74 Mol) 4-Isopropylanilin in 300 ml Eisessig werden während 3 Stunden zum Rückfluss erhitzt. Das Reaktionsgemisch wird zweimal unter verminderten Druck auf 50 ml eingeengt und dann mit Toluol unter vermindertem Druck destilliert, um die letzten Spuren der Säure zu entfernen. Das erhaltene dunkle Öl wird mit 500 ml Hexan verdünnt und zum Siedepunkt erhitzt. Um vollständige Lösung zu erhalten, werden 10 ml Äthylacetat dem siedenden Gemisch beigefügt. Beim Abkühlen des Reaktionsgemisches kristallisiert ein Festkörper aus, welcher abfiltriert, mit 100 ml Hexan gewaschen, trocken gepresst und aus 500 ml Hexan und 50 ml Äthylacetat umkristallisiert wird, wobei man 76,5 g Kristalle vom Schmelzpunkt 89-96° erhält. Eine weitere Kristallisation aus 400 ml Hexan und 100 ml Äthylacetat liefert 61,1 g (47%) 4-Isopropylacetanilid, Schmelzpunkt 103-104°.

*Beispiel 2*

Eine Suspension von 61,1 g (0,345 Mol) 4-Isopropylacetanilid und 40 g (0,49 Mol) wasserfreies Natriumacetat in 180 ml Eisessig wird in einen 1000-ml-Dreihalskolben gegeben, der durch eine geeignete

Folie vor Licht geschützt ist. Der Kolben wird mit einem Thermometer und einem Rührwerk versehen. 23,0 ml (0,45 Mol) Brom werden unter Rühren tropfenweise zugegeben. Die Zugabegeschwindigkeit wird so reguliert, dass die Temperatur des Reaktionsgemisches zwischen 40 und 50° liegt (Dauer der Zugabe 30 Minuten). Durch Dünnschichtchromatographie wird nach 30minütigem Rühren das Ende der Reaktion bestimmt (Äther/Hexan, 3:1). Das Reaktionsgemisch wird filtriert, und der Filterrückstand mit 100 ml Methylenchlorid gewaschen. Das Filtrat wird unter vermindertem Druck bei 45° zu einem gelben Festkörper eingeengt. Dieser wird in 600 ml heissem 50%igem wässrigem Äthanol gelöst. 125 ml Wasser werden zu der stark siedenden Lösung gegeben, wobei Argon kontinuierlich durch die Lösung geblasen wird. Danach wird die Flasche verschlossen und über Nacht bei 0° stehengelassen. Das Produkt wird unter Argon abfiltriert, unter vermindertem Druck (1 mm Hg) 18 Stunden bei 23° getrocknet, wobei man 88,1 g (99%) 2-Brom-4-isopropylacetanilid erhält, Schmelzpunkt 128-130°.

*Beispiel 3*

Ein Autoklav, versehen mit einem Magnetrührer, wird in 8 mg trans-Dichlor-bis-(triphenylphosphin)-palladium (II), 40 mg Triphenylphosphin und 2,0 g 2-Brom-4-isopropylacetanilid beladen. Das Gefäss wird verschlossen und dreimal mit Argon evakuiert und wieder gefüllt. Dann werden 2,2 ml Tri-n-butylamin und 0,5 ml Wasser zugegeben. Die Flasche wird mit 3 Atm. Kohlenmonoxyd beladen und verschlossen. Das Reaktionsgemisch wird während 18 Stunden auf eine Badtemperatur von 120-125° erwärmt. Nach dem Abkühlen auf 23° beträgt der Druck 1,14 Atm. Die Flasche wird entlüftet und geöffnet. Dünnschichtchromatographie (Laufmittel 50 ml Äthylacetat, 75 ml Chloroform und 5 Tropfen Essigsäure) bestätigt, dass das gesamte Bromacetanilid reagiert hat. Die erhaltene Mischung ist ein viskoses orange-braunes Öl. Zu der Mischung werden 8 ml 2,4M Salzsäure in 2-ml-Portionen gegeben, wobei man konstant rührt, bis das Reaktionsgemisch zu einer klaren Lösung und einem ausgefallenen Produkt reagiert hat. Das Produkt wird abfiltriert, mit Wasser gewaschen und unter vermindertem Druck (1 mm Hg) 18 Stunden bei 23° getrocknet. Die Ausbeute an 2-Acetylamino-5-isopropylbenzoesäure beträgt 1,63 g (94%), welche gemäss NMR-Spektrum eine Reinheit von < 95% aufweist. Die rohe Säure wird aus Äthanol/Wasser umkristallisiert, wobei man 1,35 g eines leicht gelblichen Festkörpers vom Schmelzpunkt 167-170,5° erhält.

*Beispiel 4*

Zu 10 ml einer 10%igen wässrigen Lösung von Natriumhydroxyd wird 1,0 g 2-Acetylamino-5-isopropylbenzoesäure gegeben. Die Lösung wird während 3 Stunden zum Rückfluss erhitzt. Die Beendigung der Reaktion wird dünnschichtchromatographisch bestimmt. Man lässt das Reaktionsgemisch abkühlen, wäscht es zweimal mit Methylenchlorid und stellt mit Essigsäure den pH-Wert auf 5 ein. Das Produkt wird mit zweimal 20 ml Äthylacetat extrahiert. Die vereinigten Äthylacetatextrakte werden mit 10 ml Wasser und 10 ml gesättigter Natriumchloridlösung gewaschen, über Magnesiumsulfat getrocknet, abfiltriert und zu einem Öl eingeengt. Das Produkt wird in 10 ml heissem Heptan gelöst und angeimpft, wobei man 0,65 g Kristalle erhält. Das Produkt wird dann aus 8 ml Heptan umkristallisiert, wobei man 2-Amino-5-isopropylbenzoesäure vom Schmelzpunkt 128-131° erhält. Eine zweite Umkristallisation aus Methylenchlorid/Hexan liefert ein Produkt vom Schmelzpunkt 130-131°.

*Beispiel 5*

Ein 300-ml-Autoklav wird unter einer Inertgasatmosphäre mit 370 mg trans-Dichlor-bis-(triphenylphosphin)palladium, 1,8 g Triphenylphosphin, 88,8 g (0,35 Mol) 2-Brom-4-isopropylacetanilid, 97,7 ml Tri-n-butylamin und 22,2 ml Wasser beladen. Das Reaktionsgemisch wird danach bei 23° mit etwa 3 Atm. Kohlenmonoxyd beladen und bei einer Temperatur von 115-120° während 22 Stunden umgesetzt. Soweit nötig wird Kohlenmonoxyd nachgegeben. Das Reaktionsgemisch, ein viskoses Öl, wird mit insgesamt 900 ml einer 10%igen wässrigen Natriumhydroxydlösung unter Argon aus dem Reaktionsgefäss herausgespült, welches danach mit dreimal 10 ml 95%igem Äthanol gespült wird. Die kombinierten Waschlösungen werden unter Rühren während 18 Stunden auf ungefähr 95° erwärmt (Innentemperatur). Man lässt das Reaktionsgemisch abkühlen und extrahiert das Zweiphasensystem mit dreimal 100 ml sauerstofffreiem Methylenchlorid. Die kombinierten Methylenchloridextrakte werden mit 100 ml einer sauerstofffreien 10%igen wässrigen Natriumhydroxydlösung gewaschen. Der pH-Wert der wässrigen Lösungen wird dann auf 3,0-3,5 eingestellt, und zwar zunächst durch die langsame Zugabe von ungefähr 100 ml konzentrierter Salzsäure (bis pH 5) und dann durch Titrieren mit 2,4M Salzsäure. Die angesäuerten wässrigen Lösungen werden mit drei 200-ml-Portionen Äthylacetat extrahiert, über Magnesiumsulfat getrocknet, filtriert und unter vermindertem Druck eingedampft, wobei man rohe kristalline Aminosäure erhält. Nach dem 18stündigen Trocknen bei 23° unter vermindertem Druck (1 mm Hg) wiegt die trockene Säure 53,4 g. Diese wird in ungefähr 180 ml siedendem sauerstofffreiem Methylenchlorid gelöst, und dann werden ungefähr 180 ml sauerstofffreies Hexan zugegeben. Das Produkt kristallisiert bei 0° aus. Die umkristallisierte Säure wird unter Argon abfiltriert, mit Hexan gewaschen und während 18 Stunden bei 23° und 0,1 mm Hg getrocknet. Die Ausbeute an leicht gelblich kristalliner 2-Amino-5-isopropylbenzoesäure mit dem Schmelzpunkt 130-132° beträgt 34,2 g (72%). Das Filtrat wird unter vermindertem Druck eingedampft und der Rückstand in 30 ml siedendem Methylenchlorid gelöst. Dazu werden 60 ml Hexan gegeben und das Gemisch erhitzt bis es trübe wird. Die Mischung wird dann angeimpft und bei 0° stehengelassen. Zusätzliche 7 g brauner Kristalle vom Schmelzpunkt 125-128° werden so erhalten. Gemäss $^1$H-NMR-Analyse beträgt die Reinheit der ersten Fraktion ungefähr 97% und die der zweiten Fraktion ungefähr 95%.

*Beispiele 6-13*

In ähnlicher Weise wie in den vorhergehenden Beispielen beschrieben, wird eine Serie von Ansätzen zur Herstellung von 2-Amino-5-isopropylbenzoesäure ausgeführt. Die Ansätze werden so durchgeführt, dass man zunächst 2-Brom-4-isopropylacetanilid mit Kohlenmonoxyd in Gegenwart eines Palladiumtriphenylphosphinkomplexes, Triphenylphosphin, Wasser und Tributylamin bei ungefähr 125° und einem Druck von 3-15 Atm. umsetzt. Das erhaltene Produkt, nämlich die 2-Acetylamino-5-isopropylbenzoesäure, wird mit 10%iger wässriger Natriumhydroxydlösung bei 90° während 18 Stunden hydrolysiert. Einzelheiten über den verwendeten Katalysator sowie das Gewichtsverhältnis von Substrat, d.h. 2-Brom-4-isopropylacetanilid, und Katalysator (S/K-Verhältnis) ist in der nachfolgenden Tabelle für jeden Ansatz angegeben. Im weiteren sind in der Tabelle auch die Mengen an verwendetem Ausgangsmaterial, nämlich 2-Brom-4-isopropylacetanilid, die jeweilige Reaktionsdauer für die Carbonylierungsreaktion und die Ausbeuten angegeben. Beispiele 6-10 werden in einer Fischer-Porter-Flasche, in ähnlicher Weise wie im Beispiel 3 angegeben, und die Beispiele 11-13 in einem Autoklaven, in ähnlicher Weise wie in Beispiel 5 beschrieben, durchgeführt.

| Beispiel | Katalysator | S/K | Ausgangsmaterial in g | Reaktionsdauer in Stunden | Ausbeute[a] in % |
|---|---|---|---|---|---|
| 6 | $Pd(PPh_3)_2Cl_2$ | 250 | 2 | 8 | 94[b] |
| 7 | $Pd(PPh_3)_2Cl_2$ | 500 | 2 | 18 | 75 |
| 8 | $Pd(PPh_3)_2Cl_2$ | 1000 | 2 | 36 | 79 |
| 9 | $Pd(PPh_3)_2Br_2$ | 250 | 2 | 8,5 | 84[b] |
| 10 | $Pd(PPh_3)_4$ | 250 | 2 | 10 | 79 |
| 11 | $Pd(PPh_3)_2Cl_2$ | 250 | 10 | 6,5 | 82[c] |
| 12 | $Pd(PPh_3)_2Cl_2$ | 250 | 200 | 7 | 70 |
| 13 | $Pd(PPh_3)_2Cl_2$ | 250 | 400 | 1,5 | 70 |

a Ausbeute nach Umkristallisation (1 Fraktion)

b Ausbeute an 2-Acetylamino-5-isopropylbenzoesäure

c Ausbeute aus zwei Fraktionen

*Beispiel 14*

Eine Fischer-Porter-Flasche, bestückt mit einem Magnetrührer, wird unter einer Inertgasatmosphäre mit 1,0 g 2-Brom-4-methoxyacetanilid, 0,01 g trans-Dichlor-bis-(triphenylphosphin)palladium und 0,01 g Triphenylphosphin beladen. Das Gefäss wird verschlossen, evakuiert und dreimal mit Argon wieder gefüllt. Dann werden 1,0 ml Tri-n-butylamin und 0,3 ml Wasser zugegeben. Die Flasche wird dann mit Kohlenmonoxyd auf 2,7 Atm. beladen und verschlossen. Das Reaktionsgemisch wird während 18 Stunden bei einer Maximalbadtemperatur von 125° erhitzt. Dann lässt man abkühlen, entlüftet und öffnet die Flasche. Zum Rohprodukt werden 3 ml einer 2N Salzsäurelösung gegeben. Das Gemisch wird filtriert, mit Wasser gewaschen und unter vermindertem Druck (1 mm Hg) während 18 Stunden bei 20° getrocknet. Das so erhaltene Produkt (77%) ist ein leicht oranger Festkörper. Dieses Produkt wird in Äthanol gelöst, filtriert, und die Lösung erhitzt. Dann wird Wasser zu der heissen Lösung gegeben, bis diese leicht trübe wird. Die Lösung wird dann nochmals filtriert, um etwas ausgefallenen roten Festkörper zu entfernen, und dann abgekühlt. Der leicht gelbliche Niederschlag wird abfiltriert und während 4 Stunden bei 20° unter vermindertem Druck bei 1 mm Hg getrocknet. Auf diese Weise erhält man 0,235 g 2-Acetylamino-5-methoxybenzoesäure, Schmelzpunkt 160-161°.

2,5 g so erhaltener Acetylaminosäure werden hydrolysiert, indem man sie in einem Gemisch von 15 ml Wasser, 5 ml Äthanol und 4 ml 5M Natriumhydroxydlösung während 18 Stunden unter Argon zum Rückfluss erhitzt, danach weitere 5 ml 5M Natriumhydroxydlösung zugibt und weitere 24 Stunden zum Rückfluss erhitzt. Das Reaktionsgemisch lässt man abkühlen, extrahiert mit dreimal 10 ml Methylenchlorid, säuert mit ungefähr 3 ml Essigsäure an und extrahiert nochmals mit dreimal 20 ml Methylenchlorid. Die letztgenannten Methylenchloridextrakte werden über Magnesiumsulfat getrocknet, filtriert und eingedampft, wobei man 0,91 g (46%) 2-Amino-5-methoxybenzoesäure erhält, Schmelzpunkt 145-148°. Umkristallisation aus Methylenchlorid/Hexan liefert 0,52 g eines Produkts, welches ein zweites Mal kristallisiert wird, wobei man 0,09 g eines Produkts vom Schmelzpunkt 151-153°, und aus der Mutterlauge 0,37 g eines Produkts vom Schmelzpunkt 150-152° erhält.

*Beispiel 15*

Zu einer auf 15° abgekühlten Lösung von 97,9 g (0,73 Mol) Isobutylbenzol in 400 ml Essigsäure und 100 ml Essigsäureanhydrid werden 41 ml (1,0 Mol) 90%iger salpetriger Säure unter Rühren innerhalb von 2 Stunden gegeben. Das Reaktionsgemisch wird dann während einer weiteren Stunde bei 15° gerührt, vom Eisbad entfernt und während weiteren 2 Stunden bei Raumtemperatur gerührt. 1000 ml Wasser werden zugegeben und das Produkt dreimal mit Methylenchlorid extrahiert. Die kombinierten Extrakte werden mit Wasser, zweimal mit 1N Natriumhydroxydlösung und wiederum mit Wasser gewaschen, über Magnesiumsulfat getrocknet und eingedampft, wobei man 124 g eines Öls, bestehend aus einer Mischung von o-Isobutylnitrobenzol und p-Isobutylnitrobenzol erhält. Durch fraktionierte Destillation unter Verwendung einer Vigreux-Kolonne und

danach einer Goodloe-Kolonne werden 35 g eines Produkts erhalten, welches überwiegend aus p-Isobutylnitrobenzol besteht, zusammen mit etwas o-Isobutylnitrobenzol. Dieses Produkt wird in 150 ml Äthylacetat gelöst und über 0,5 g Palladium auf Kohle und einem Wasserstoffdruck von 3,6 Atm. hydriert. Das gebildete p-Isoputylaminobenzol wird durch Celit® filtriert und zu einem Öl eingedampft. Danach werden 100 ml Essigsäure zugegeben, und das Gemisch während 3 Stunden und 20 Minuten zum Rückfluss erhitzt. Das erhaltene Produkt wird zu einem Festkörper eingedampft, mit Hexan angerieben und abfiltriert, wobei man 28,65 g p-Isobutylacetanilid erhält. Ein Öl, welches von diesem Produkt abgetrennt wird, wird zur Trockne eingedampft, mit Hexan angerieben, abfiltriert und mit dem übrigen Produkt vereinigt. Das erhaltene Rohprodukt wird aus Äthylacetat/Hexan umkristallisiert, wobei man 23,42 g eines Produkts vom Schmelzpunkt 116 bis 118° erhält. Eine zweite Fraktion von 2,65 g (Schmelzpunkt 108-111°) wird bei der Umkristallisation aus Äthylacetat/Hexan erhalten.

Zu einer Suspension von 25,8 g (0,135 Mol) p-Isobutylacetanilid und 15,6 g (0,19 Mol) wasserfreiem Natriumacetat in 80 ml Essigsäure werden tropfenweise unter Rühren 9 ml Brom gegeben, wobei sich der Festkörper nach und nach auflöst. Die Zugabe wird bei 28-30° durchgeführt und dauert 25 Minuten. Nach 40 Minuten bildet sich ein Festkörper und das Rühren wird während 2³/₄ Stunden fortgesetzt. Das Reaktionsgemisch wird zur Entfernung des Niederschlags filtriert, und das Filtrat unter vermindertem Druck auf ein kleines Volumen eingeengt. Danach gibt man Wasser zu und filtriert das Gemisch, um den ausgefallenen Festkörper abzutrennen. Das Produkt wird mit verdünnter wässriger Natriumsulfitlösung gewaschen und dann an der Luft getrocknet. Umkristallisation aus Äthanol/Wasser liefert 29,7 g 2-Brom-4-isobutylacetanilid.

*Beispiel 16*

Eine Fischer-Porter-Flasche wird mit 10 mg trans-Dichlor-bis-(triphenylphosphin)palladium, 30 mg Triphenylphosphin und 1,0 g 2-Brom-4-isobutylacetanilid beladen. Das Gefäss wird verschlossen, evakuiert und mit Argon wieder gefüllt. Dann werden 1 ml Tri-n-butylamin und 0,35 ml Wasser zugegeben. Die Flasche wird dann mit 2,9 Atm. Kohlenmonoxyd beladen und verschlossen. Man lässt während 18 Stunden bei einer Reaktionstemperatur von 125° reagieren. Nach beendeter Reaktion beträgt der Druck in der Flasche 1,14 Atm. bei 23°. Die Flasche wird entlüftet und geöffnet. Dünnschichtchromatographie zeigt an, dass die Reaktion vollständig ist.

Das Rohprodukt, welches 2-Acetylamino-5-isobutylbenzoesäure enthält, wird in einen 25-ml-Rundkolben transferiert, in Äthanol gelöst und unter vermindertem Druck eingedampft. Dann werden 10 ml einer 3N Natriumhydroxydlösung und 0,5 ml Äthanol zugegeben. Die Reaktionslösung wird während 18 Stunden zum Rückfluss erhitzt. Dann gibt man 10 ml Wasser zu, wäscht das Zweiphasensystem mit dreimal 10 ml Methylenchlorid und bringt danach die wässrige Phase durch Zugabe von 6N Salzsäure auf einen pH-Wert von 3,25, wobei ein leicht gelblicher Festkörper ausfällt. Die Suspension wird dreimal mit 25 ml Äthylacetat extrahiert, die vereinigten Äthylacetate über Magnesiumsulfat getrocknet, filtriert und eingedampft, wobei man 715 mg eines gelben Festkörpers erhält. Das Rohprodukt wird aus Methylenchlorid/Hexan umkristallisiert, wobei man 606 mg (85%) 2-Amino-5-isobutylbenzoesäure als leicht gelben Festkörper erhält, Schmelzpunkt 124-127°.

*Beispiel 17*

60 g sec.-Butylnitrobenzol (vorwiegend das para-Isomere) werden in 200 ml Äthylacetat gelöst und in Gegenwart von 1,0 g Palladium auf Kohle unter einem Wasserstoffdruck von ungefähr 3,6 Atm. während 6 Stunden hydriert. Das rohe Reaktionsprodukt wird durch Celit filtriert und zu 48,95 g sec-Butylanilin eingedampft.

Eine Mischung von 44 g sec.-Butylanilin und 135 ml Eisessig wird während 3 Stunden zum Rückfluss erhitzt, wobei man eine braune Lösung erhält. Das Produkt wird unter vermindertem Druck eingedampft. Dann werden zweimal je 50 ml Toluol zugegeben, und danach wird das Reaktionsgemisch eingedampft, wobei man einen braunen Festkörper erhält. Das Produkt wird zweimal umkristallisiert, das erste Mal aus Äthylacetat/Hexan und danach aus Methylenchlorid/Hexan, wobei man 38 g eines braunes Festkörpers erhält, der bei 109-117° schmilzt. Eine letzte Umkristallisation aus Äthylacetat/Hexan liefert 34,8 g (62%) sec-Butylacetanilid als leicht gelblichen Festkörper, Schmelzpunkt 110-116°.

Zu einer Suspension von 34,8 g sec-Butylacetanilid in 115 ml Eisessig und 20,9 g wasserfreiem Natriumacetat werden 12,2 ml Brom tropfenweise während 30 Minuten gegeben. Die Reaktionstemperatur steigt von 29° auf 38° an. Das Reaktionsgemisch wird über Nacht gerührt und danach unter vermindertem Druck eingedampft. Man gibt 500 ml einer gesättigten Natriumbicarbonatlösung zum Rückstand, rührt das Gemisch während 15 Minuten und filtriert, wobei man einen braunen Festkörper erhält. Der braune Festkörper wird in 500 ml Methylenchlorid gelöst, zweimal mit je 250 ml einer gesättigten Natriumbicarbonatlösung und mit 250 ml Wasser gewaschen. Die organische Phase wird zu einem braunen Festkörper eingedampft, welcher aus Äthanol/Wasser umkristallisiert und zweimal gewaschen wird, wobei man 31,0 g 2-Brom-4-sec.-butylacetanilid als leicht gelblichen Festkörper erhält, Schmelzpunkt 89,5-92°. Eine kleine Menge an weiterem Festkörper wird aus der Mutterlauge isoliert, abfiltriert und gewaschen, wobei man 4,5 g eines Produkts vom Schmelzpunkt 86-88° erhält.

*Beispiel 18*

Eine Fischer-Porter-Flasche mit Magnetrührer wird unter einer Inertgasatmosphäre mit 1,0 g 2-Brom-4-sec.-butylacetanilid, 0,01 g trans-Dichlor-bis-(triphenylphosphin)palladium und 0,03 g Triphenylphosphin, 1,0 ml Tri-n-butylamin und 0,35 ml Wasser beladen. Die Flasche wird mit 2,8 Atm. Kohlenmonoxyd beladen und verschlossen. Das Reaktionsgemisch wird während 18 Stunden unter einem Druck von 2,15-3,6 Atm. Kohlenmonoxyd auf 125° erwärmt und nachher abgekühlt. Basische Hydrolyse

des rohen Reaktionsgemisches und Aufarbeitung liefert 0,77 g (88%) an umkristallisierter 2-Amino-5--sec.-butylbenzoesäure.

*Beispiel 19*

Eine Fischer-Porter-Flasche, mit Magnetrührer versehen, wird mit 1,0 g 2-Brom-4,6-diisopropylacetanilid, 0,01 g trans-Dichlor-bis-(triphenylphosphin)palladium und 0,01 g Triphenylphosphin beladen. Das Gefäss wird verschlossen, dreimal evakuiert und wieder mit Argon gefüllt. Dann werden 1,0 ml Tri-n-butylamin und 0,35 ml Wasser zugegeben. Die Flasche wird mit 2,8 Atm. Kohlenmonoxyd beladen und verschlossen. Das Reaktionsgemisch wird dann auf eine maximale Badtemperatur von 124° während $16^1/_2$ Stunden erhitzt und dann abgekühlt. Der Druck nach beendeter Reaktion beträgt 2 Atm. bei 20°. Hydrolyse und Aufarbeiten liefert 0,57 g (69%) 2-Amino-3,5-diisopropylbenzoesäure.

**Patentansprüche**

1. Verfahren zur Herstellung von Anthranilsäurederivaten der allgemeinen Formel

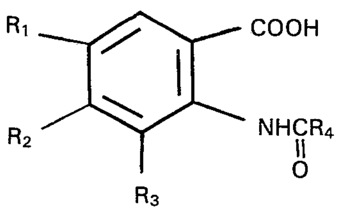

I

worin $R_1$, $R_2$ und $R_3$ unabhängig voneinander je Wasserstoff, niederes Alkyl, niederes Alkoxy, Carboxyl, Chlor, Fluor oder gegebenenfalls durch niederes Alkyl oder niederes Alkoxy substituiertes Phenyl und $R_4$ niederes Alkyl oder Phenyl bedeuten, dadurch gekennzeichnet, dass man unter im wesentlichen sauerstofffreien Bedingungen Kohlenmonoxyd in einem wässrigen Reaktionsmedium, welches ein Trialkylamin und einen Katalysator, gebildet aus Palladium und einem tertiären Phosphin, enthält, mit einer Verbindung der allgemeinen Formel

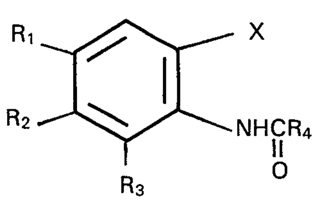

II

worin X Brom oder Jod bedeutet und $R_1$, $R_2$, $R_3$ und $R_4$ die oben angegebene Bedeutung besitzen, umsetzt.

2. Verfahren nach Anspruch 1, dadurch gekennzeichnet, dass man ein Ausgangsmaterial der Formel II verwendet, worin $R_1$ niederes Alkyl oder niederes Alkoxy, $R_2$ und $R_3$ je Wasserstoff und $R_4$ niederes Alkyl bedeuten.

3. Verfahren nach Anspruch 1 oder 2, dadurch gekennzeichnet, dass das Molverhältnis von tertiärem Phosphin zu Palladium im Katalysator mindestens 2:1 beträgt.

4. Verfahren nach einem der Ansprüche 1-3, dadurch gekennzeichnet, dass das molare Verhältnis an Ausgangsmaterial der Formel II zu Katalysator basierend auf der Molmenge an Palladium im Katalysator 100:1 bis 10 000:1 beträgt.

5. Verfahren nach Anspruch 4, dadurch gekennzeichnet, dass das Verhältnis 250:1 bis 3000:1 beträgt.

6. Verfahren nach einem der Ansprüche 1-5, dadurch gekennzeichnet, dass das tertiäre Phosphin Triphenylphosphin ist.

7. Verfahren nach einem der Ansprüche 1-6, dadurch gekennzeichnet, dass man die erhaltene Verbindung der Formel I in Gegenwart einer Base zu einer Verbindung der allgemeinen Formel

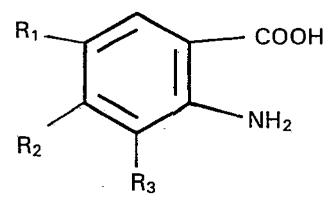

III

worin $R_1$, $R_2$ und $R_3$ unabhängig voneinander je Wasserstoff, niederes Alkyl, niederes Alkoxy, Carboxyl, Chlor, Fluor oder gegebenenfalls durch niederes Alkyl oder niederes Alkoxy substituiertes Phenyl bedeuten,
hydrolisiert.

**Claims**

1. Process for the manufacture of anthranilic acid derivatives of the general formula

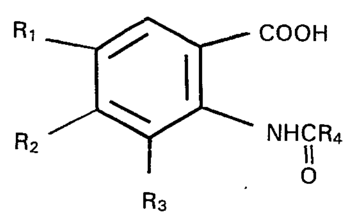

I

wherein $R_1$, $R_2$ and $R_3$ independently of one another each signify hydrogen, lower alkyl, lower alkoxy, carboxyl, chlorine, fluorine or phenyl optionally substituted by lower alkyl or lower alkoxy and $R_4$ signifies lower alkyl or phenyl,
characterized by reacting carbon monoxide under essentially oxygen-free conditions in an aqueous reaction medium which contains a trialkylamine and a catalyst formed from palladium and a tertiary phosphine with a compound of the general formula

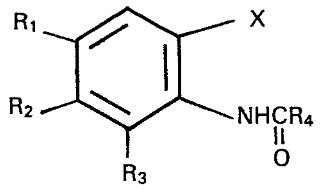

II

wherein X signifies bromide or iodine and $R_1$, $R_2$, $R_3$ and $R_4$ have the significance given above.

2. Process according to claim 1, characterized in that a starting material of formula II wherein $R_1$ signifies lower alkyl or lower alkoxy, $R_2$ and $R_3$ each signify hydrogen and $R_4$ signifies lower alkyl is used.

3. Process according to claim 1 or 2, characterized in that the molar ratio of tertiary phosphine to palladium in the catalyst is at least 2:1.

4. Process according to any one of claims 1-3, characterized in that the molar ratio of starting material of formula II to catalyst is 100:1 to 10 000:1 based on the molar amount of palladium in the catalyst.

5. Process accoring do claim 4, characterized in that the ratio is 250:1 to 3000:1.

6. Process according to any one of claims 1-5, characterized in that triphenylphosphine is the tertiary phosphine.

7. Process according to any one of claims 1-6, characterized in that the compound of formula I obtained is hydrolyzed in the presence of a base to a compound of the formula

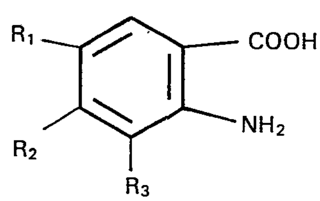

III

wherein $R_1$, $R_2$ and $R_3$ independently of one another each signify hydrogen, lower alkyl, lower alkoxy, carboxyl, chlorine, fluorine or phenyl optionally substituted by lower alkyl or lower alkoxy.

**Revendications**

1. Procédé de préparation de dérivés d'acide anthranilique de formule générale

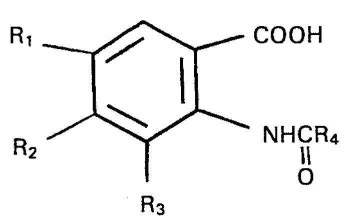

I

où $R_1$, $R_2$ et $R_3$ représentent chacun indépendamment l'un de l'autre un hydrogène, un alcoyle inférieur, un alcoxy inférieur, un carboxyle, un chlore, un fluor ou un phényle éventuellement substitué par un alcoyle inférieur ou un alcoxy inférieur et $R_4$ représente un alcoyle inférieur ou un phényle,
caractérisé en ce qu'on fait réagir dans des conditions pratiquement dépourvues d'oxygène, du monoxyde de carbone dans un milieu réactionnel aqueux, qui contient une trialcoylamine et un catalyseur formé à partir de palladium et d'une phosphine tertiaire, avec un composé de formule générale

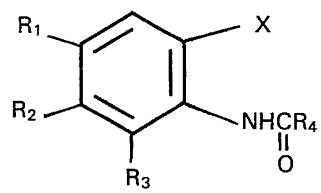

II

où X représente un brome ou un iode et $R_1$, $R_2$, $R_3$ et $R_4$ ont les significations données ci-dessus.

2. Procédé selon la revendication 1, caractérisé en ce qu'on utilise un produit de départ de formule II où $R_1$ représente un alcoyle inférieur ou un alcoxy inférieur, $R_2$ et $R_3$ représentent chacun un hydrogène et $R_4$ représente un alcoyle inférieur.

3. Procédé selon l'une des revendications 1 et 2, caractérisé en ce que le rapport molaire de la phosphine tertiaire au palladium dans le catalyseur s'élève au moins à 2:1.

4. Procédé selon l'une des revendications 1-3, caractérisé en ce que le rapport molaire du produit de départ de formule II au catalyseur sur la base de la quantité molaire de palladium dans le catalyseur s'élève à 100:1 à 10 000:1.

5. Procédé selon la revendication 4, caractérisé en ce que le rapport s'élève à 250:1 à 3000:1.

6. Procédé selon l'une des revendications 1-5, caractérisé en ce que la phosphine tertiaire est la triphénylphosphine.

7. Procédé selon l'une des revendications 1-6, caractérisé en ce qu'on hydrolyse le composé de formule I obtenu en présence d'une base en un composé de formule générale

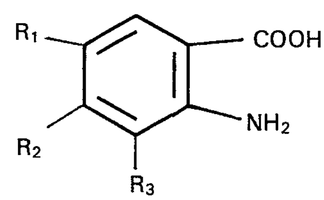

III

où $R_1$, $R_2$ et $R_3$ représentent chacun indépendamment l'un de l'autre un hydrogène, un alcoyle inférieur, un alcoxy inférieur, un carboxyle, un chlore, un fluor ou un phényle éventuellement substitué par un alcoyle inférieur ou un alcoxy inférieur.